# EUROPEAN PATENT SPECIFICATION

(11) **EP 2 150 613 B1**
(45) Date of publication and mention of the grant of the patent: **19.10.2016**
(21) Application number: 08748334.3
(22) Date of filing: 21.05.2008
(51) Int. Cl.: C12N 7/01, A61K 35/76, A61P 35/00, C07K 14/14, C12N 15/86, C12N 15/46

(54) **MUTANT REOVIRUSES AND METHODS OF MAKING AND USING**
MUTANTE REOVIREN SOWIE VERFAHREN ZU IHRER HERSTELLUNG UND VERWENDUNG
RÉOVIRUS MUTANTS ET LEURS PROCÉDÉS DE FABRICATION ET D'UTILISATION

(30) Priority: 21.05.2007 US 939255 P
(43) Date of publication of application: 10.02.2010
(73) Proprietor: Oncolytics Biotech, Inc., Calgary, Alberta T2N 1X7 (CA)
(72) Inventor: COFFEY, Matthew C., Calgary, Alberta T2N 3L4 (CA); THOMPSON, Bradley G., Calgary, Ontario T2N 0Z5 (CA)
(74) Representative: Nash, David Allan
(86) International application number: PCT/CA2008/000964
(87) International publication number: WO 2008/141448

(56) References cited:
- WO-A1-2005/111200
- WO-A2-2007/099401
- US-B2- 7 163 678
- EBERT D.H. ET AL.: 'Adaptation of reovirus to growth in the presence of protease inhibitor E64 segregates with a mutation in the carboxy terminus of viral outer-capsid protein sigma3' J. VIROLOGY vol. 75, no. 7, April 2001, pages 3197 - 3206, XP002477299
- CLARK K. ET AL.: 'Reovirus variants selected for resistance to ammonium chloride have mutations in viral outer-capsid protein sigma3' J. VIROLOGY vol. 80, no. 2, January 2006, pages 671 - 681, XP008117574
- WETZEL J.D. ET AL.: 'Reovirus variants selected during persistent infections of L cells contain mutations in the viral S1 and S4 genes and are altered in viral disassembly' J. VIROLOGY vol. 71, no. 2, February 1997, pages 1362 - 1369, XP008117575

## Description

### BACKGROUND

The ability of reovirus to establish a productive infection requires proteolysis of the outer capsid. Proteolysis converts the virion into a form referred to as an intermediate subvirion particle (ISVP), which has the capacity to penetrate cell membranes and thereby gain access to cytoplasmic components required for viral gene expression. Partial proteolysis of an intact reovirus virion to generate an ISVP also can be performed *in vitro* using a protease such as chymotrypsin or trypsin. See, for example, Golden et al. (2002, J. Virol., 76:7430-43) and Chandran & Nibert (1998, J. Virol., 72:467-75).

### SUMMARY

The present invention is defined in and by the appended claims. This disclosure describes mutant reoviruses that lack or exhibit reduced expression of the sigma3 polypeptide or that lack a functional sigma3 polypeptide. The mutant reoviruses described herein can be used to generate and stably propagate ISVPs.

A mutant reovirus is described that comprises a first mutation that reduces expression of a sigma3 polypeptide, essentially eliminates expression of a sigma3 polypeptide, or results in an absence of a functional sigma3 polypeptide. The first mutation can be in a nucleic acid encoding the sigma3 polypeptide or in a nucleic acid that regulates expression of the sigma3 polypeptide. In certain instances, the nucleic acid is the S4 gene. Representative mutations include a genetically-engineered substitution and a genetically-engineered insertion or deletion of one or more nucleotides. Typically, the first mutation reduces expression of the sigma3 polypeptide by at least 30%.

A mutant reovirus as described herein can include one or more further mutations. The further mutation or mutations can be a mutation that reduces expression of a mu1 polypeptide, essentially eliminates expression of a mu1 polypeptide, or results in an absence of a functional mu1 polypeptide; a mutation that reduces expression of a lambda2 polypeptide, essentially eliminates expression of a lambda2 polypeptide, or results in an absence of a functional lambda2 polypeptide; and/or a mutation that reduces expression of a sigma1 polypeptide, essentially eliminates expression of a sigma1 polypeptide, or results in an absence of a functional sigma1 polypeptide.

A genetically-engineered reovirus infectious subviral particle (ISVP) also is provided. Such a reovirus ISVP can be genetically-engineered to exhibit reduced expression of a sigma3 polypeptide, to lack expression of a sigma3 polypeptide, or to express a non-functional sigma3 polypeptide. A reovirus ISVP as described herein can be genetically-engineered to include a first mutation. The first mutation can be in a nucleic acid encoding the sigma3 polypeptide or in a nucleic acid that regulates expression of the stigma3 polypeptide. In certain instances, the nucleic acid is the S4 gene. Representative mutations include a substitution and an insertion or deletion of one or more nucleotides. Typically, the first mutation reduces expression of the sigma3 polypeptide by at least 30%.

A mutant reovirus as described herein can include one or more further mutations. The further mutation or mutations can be a mutation that reduces expression of a mul polypeptide, essentially eliminates expression of a mul polypeptide, or results in an absence of a functional mu1 polypeptide; a mutation that reduces expression of a lambda2 polypeptide, essentially eliminates expression of a lambda2 polypeptide, or results in an absence of a functional lambda2 polypeptide; and/or a mutation that reduces expression of a sigma1 polypeptide, essentially eliminates expression of a sigma1 polypeptide, or results in an absence of a functional sigma1 polypeptide.

A method of making a reovirus having increased infectivity is provided. Such a reovirus can be made by mutating a first reovirus to generate a mutant reovirus, wherein the mutant reovirus exhibits reduced expression of a sigma3 polypeptide, lacks expression of a sigma3 polypeptide, or expresses a non-functional stigma3 polypeptide and isolating the mutant reovirus, wherein the mutant reovirus has increased infectivity compared to the first reovirus. Increased infectivity can be evidenced by an increase in the range of neoplastic cells that can be infected by the mutant reovirus compared to the first reovirus or by an increase in the number of cells infected by the mutant reovirus compared to the first reovirus.

A method of obtaining a genetically-engineered reovirus infectious subviral particle (ISVP) also is provided. Such a method includes genetically-engineering a first reovirus to exhibit reduced expression of a sigma3 polypeptide, to lack expression of a stigma3 polypeptide, or to express a non-functional sigma3 polypeptide; culturing the genetically-engineered reovirus, and isolating ISVPs to thereby obtain a genetically-engineered reovirus ISVP.

Further provided is a method of treating a proliferative disorder in a subject. Such a method of treating includes administering, to the subject, the mutant reovirus disclosed herein or the genetically-engineered reovirus ISVP disclosed herein. Such methods also can include at least one procedure chosen from surgery, chemotherapy, radiation therapy, and immunosuppressive therapy.

A pharmaceutical composition is provided that includes the mutant reovirus described herein or the genetically-engineered reovirus ISVP described herein. Such a pharmaceutical composition further can include one or more chemotherapeutic agents and/or one or more immunosuppressive agents.

Also provided is a sigma3 polypeptide that includes a mutation that results in a sigma3 polypeptide that is not incorporated into a viral capsid or that is incorporated at reduced levels into the capsid.

Unless otherwise defined, all technical and scientific terms used herein have the same meaning as commonly understood by one of ordinary skill in the art to which the methods and compositions of matter belong. Although methods and materials similar or equivalent to those described herein can be used in the practice or testing of the present methods and compositions of matter, suitable methods and materials are described below. In addition, the materials, methods, and examples are illustrative only and not intended to be limiting.

### DESCRIPTION OF DRAWINGS

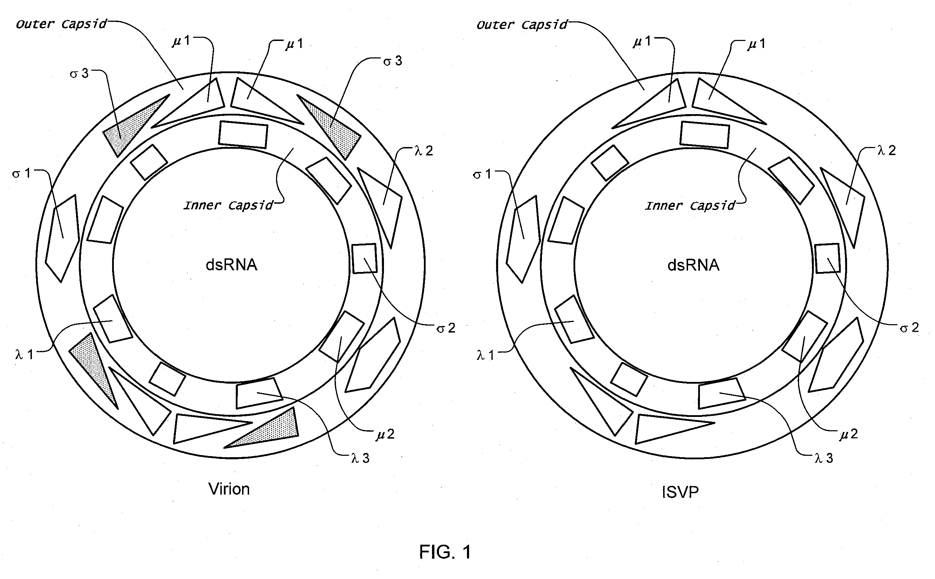

Figure 1 is a schematic showing the structural elements of a reovirus virion and a reovirus ISVP.

### DETAILED DESCRIPTION

Mutant reoviruses that lack or exhibit reduced expression of the sigma3 polypeptide or that lack a functional sigma3 polypeptide are described herein. In addition to containing a mutation in the nucleic acid encoding or regulating the sigma3 polypeptide, the mutant reoviruses described herein also can contain a further mutation in one or more of the other outer capsid proteins. The mutant reoviruses provided herein can be used to generate intermediate subvirion particles (ISVPs) that can be stably propagated as ISVPs for multiple passages. ISVPs typically exhibit increased infectivity and/or decreased immunogenicity compared to reovirus itself, but ISVPs typically lack the ability to propagate in a stable fashion. The ISVPs formed by the mutant reovirus, in contrast, demonstrate stable propagation for multiple replications, unlike ISVPs created by enzymatic treatment or the like.

### Mutant Reoviruses and Methods of Making

Mutant reoviruses as described herein can contain a first mutation that reduces or essentially eliminates expression of a sigma3 polypeptide or that results in the absence of a functional sigma3 polypeptide. A mutation that eliminates expression of a sigma3 polypeptide or that results in the absence of a functional sigma3 polypeptide can be in the nucleic acid encoding the sigma3 polypeptide (i.e., the S4 gene) or in a nucleic acid that encodes a polypeptide that regulates the expression or function of the sigma3 polypeptide.

The sigma3 polypeptide is 365 amino acids in length and has a molecular weight of 41 kDa. An intact virion contains 600 copies of the sigma3 polypeptide. The sigma3 polypeptide likely interacts with other outer capsid proteins including, for example, mu1, lambda2, and sigma1, and also may play a role in RNA selection or RNA packaging. Images of reovirus virions demonstrate that the sigma3 polypeptide projects like fingers on the surface of the virion and contributes significantly to the density of the virion. See Figure 1.

As used herein, a mutation that reduces the expression of a sigma3 polypeptide refers to a mutation that results in a decrease in the amount of sigma3 polypeptides, compared to a reovirus expressing wild type levels of sigma3 polypeptide, of at least 30% (e.g., at least 40%, 50%, 60%, 70%, 80%, 90%, or 95%). As used herein, a mutation that essentially eliminates expression of a sigma3 polypeptide refers to a mutation that results in a decrease in the amount of sigma3 polypeptides, relative to the amount of sigma3 polypeptides produced by a wild type reovirus, of at least 95% (e.g., 96%, 97%, 98%, 99%, or 100%). As used herein, a mutation that results in a decrease in or absence of a functional sigma3 polypeptide refers to a mutation that allows expression of the sigma3 polypeptide but that results in a sigma3 polypeptide that is not able to assemble or incorporate into the viral capsid. It would be understood that it may be desirable or necessary for sigma3 polypeptides to retain other functionalities (e.g., the ability to bind RNA) in order that the mutant reovirus retain the ability to propagate.

A mutation in a sigma3 polypeptide as described herein can result in a sigma3 polypeptide that is incorporated into the capsid at levels that are reduced relative to a sigma3 polypeptide that does not contain the mutation (e.g., a wild type sigma3 polypeptide). A mutation in a sigma3 polypeptide as described herein also can result in a sigma3 polypeptide that cannot be incorporated into a viral capsid. Without being bound by any particular mechanism, a sigma3 polypeptide may have reduced function or lack function due, for example, to an inability of the sigma3 polypeptide and the mu1 polypeptide to bind appropriately, or to a conformational change that reduces or prohibits incorporation of the sigma3 polypeptide into the capsid.

A mutant reovirus according to this disclosure can be a type 3 mammalian orthoreovirus. Type 3 mammalian orthoreoviruses include, without limitation, Dearing and Abney strains (T3D or T3A, respectively). See, for example, ATCC Accession Nos. VR-232 and VR-824. A mutant reovirus as described herein can contain a spontaneously-generated mutation or a genetically-engineered mutation. For example, naturally-occurring reoviruses (e.g., isolated from a source in nature such as from a patient) can be mutated or recombinant reoviruses (see, e.g., U.S. Patent No. 7,163,678) can be generated that do not express the sigma3 polypeptide or that lack a functional sigma3 polypeptide. It is noted that reoviruses that carry a mutation in the S4 gene such as those described by Clark et al. (2006, J. Virol., 80:671-81), Ebert et al. (2001, J. Virol., 75:3197-206) and Wetzel et al. (1997, J. Virol., 71:1362-9) are not part of and are excluded from the present mutants because such mutants would be considered to express a functional sigma3 polypeptide.

A mutation as referred to herein can be a substitution or an insertion or deletion of one or more nucleotides. Point mutations include, for example, single nucleotide transitions (purine to purine or pyrimidine to pyrimidine) or transversions (purine to pyrimidine or *vice versa)* and single-or multiple-nucleotide deletions or insertions. A mutation in a nucleic acid can result in one or more conservative or non-conservative amino acid substitutions in the encoded polypeptide, which may result in conformational changes or loss or partial loss of function, a shift in the reading frame of translation ("frame-shift") resulting in an entirely different polypeptide encoded from that point on, a premature stop codon resulting in a truncated polypeptide ("truncation"), or a mutation in a reovirus nucleic acid may not change the encoded polypeptide at all ("silent" or "nonsense"). See, for example, Johnson & Overington, 1993, J. Mol. Biol., 233:716-38; Henikoff & Henikoff, 1992, Proc. Natl. Acad. Sci. USA, 89:10915-19; and U.S. Patent No. 4,554,101 for disclosure on conservative and non-conservative amino acid substitutions.

Mutations can be generated in the nucleic acid of a reovirus using any number of methods known in the art. For example, site directed mutagenesis can be used to modify a reovirus nucleic acid sequence. One of the most common methods of site-directed mutagenesis is oligonucleotide-directed mutagenesis. In oligonucleotide-directed mutagenesis, an oligonucleotide encoding the desired change(s) in sequence is annealed to one strand of the DNA of interest and serves as a primer for initiation of DNA synthesis. In this manner, the oligonucleotide containing the sequence change is incorporated into the newly synthesized strand. See, for example, Kunkel, 1985, Proc. Natl. Acad. Sci. USA, 82:488; Kunkel et al., 1987, Meth. Enzymol., 154:367; Lewis & Thompson, 1990, Nucl. Acids Res., 18:3439; Bohnsack, 1996, Meth. Mol. Biol., 57:1; Deng & Nickoloff, 1992, Anal. Biochem., 200:81; and Shimada, 1996, Meth. Mol. Biol., 57:157. Other methods are used routinely in the art to modify the sequence of a protein or polypeptide. For example, nucleic acids containing a mutation can be generated using PCR or chemical synthesis, or polypeptides having the desired change in amino acid sequence can be chemically synthesized. See, for example, Bang & Kent, 2005, Proc. Natl. Acad. Sci. USA, 102:5014-9 and references therein.

In addition to a mutation that abolishes or reduces expression of the sigma3 polypeptide or that results in a non-functional or reduced-function sigma3 polypeptide, a mutant reovirus as described herein also can contain one or more further mutations (e.g., a second, third, or fourth mutation) in one of the other reovirus capsid polypeptides (e.g., mu1, lambda2, and/or sigma1). Reoviruses containing a mutation affecting the sigma3 polypeptide and, optionally, a further mutation in any or all of the other outer capsid proteins can be screened for the ability of such mutant reoviruses to infect and cause lysis of cells. For example, neoplastic cells that are resistant to lysis by wild type reovirus can be used to screen for mutant reoviruses described herein.

For example, a further mutation can reduce or essentially eliminate expression of a mu1 polypeptide or result in the absence of a functional mu1 polypeptide. The mu1 polypeptide, which is encoded by the M2 gene, is likely involved in cell penetration and may play a role in transcriptase activation. Each virion contains about 600 copies of mu1 polypeptides, which are present in the form of 1:1 complexes with sigma3 polypeptides. The mu1 polypeptide is myristolated on its N-terminus, and then the myristolated N-terminal 42 residues are cleaved off, resulting in a C-terminal fragment (mu1C). Additionally or alternatively, a further mutation can reduce or essentially eliminate expression of a lambda2 polypeptide or result in the absence of a functional lambda2 polypeptide, and/or a further mutation can reduce or essentially eliminate expression of a sigma1 polypeptide or result in the absence of a functional sigmal polypeptide. The lambda2 polypeptide is encoded by the L2 gene and is involved in particle assembly, and exhibits guanylyltransferase and methyltransferase activity. The sigmal polypeptide is encoded by the S1 gene and is involved in cell-attachment and serves as the viral hemagglutinin.

Nucleic acids from reovirus particles can be isolated using standard commercially available nucleic acid methodology. See also, for example, Schiff et al., "Orthoreoviruses and Their Replication," Ch 52, in Fields Virology, Knipe & Howley, eds., 2006, Lippincott Williams & Wilkins. As used herein, isolated nucleic acids refer to nucleic acids that are separated from other nucleic acids with which they are usually associated. Thus, an isolated nucleic acid includes, without limitation, reoviral nucleic acid that is essentially free of non-reoviral (e.g., host cell) nucleic acid, or a reoviral genomic segment that is essentially free of nucleic acid corresponding to other genomic segments. In addition, an isolated nucleic acid can include an engineered nucleic acid such as a recombinant or synthetic nucleic acid.

A mutant reovirus as described herein can be generated by reconstituting genome segments containing at least a first mutation affecting the sigma3 polypeptide, which produces an infectious subviral particle (ISVP; e.g., a genetically-engineered ISVP) using methods known in the art. See, for example, Schiff et al., "Orthoreoviruses and Their Replication," Ch 52, in Fields Virology, Knipe & Howley, eds., 2006, Lippincott Williams & Wilkins; Smith et al., 1969, Virology, 39(4):791-810; and U.S. Patent Nos. 7,186,542; 7,049,127; 6,808,916; and 6,528,305. A mutant reovirus also can be generated by expressing the reovirus genome segments using a plasmid-based reverse genetic system to produce an ISVP. See, for example, Kobayashi et al., 2007, Cell Host & Microbe, 1:147-57. As used herein, a genetically-engineered or mutant ISVP is a mutant reovirus and refers to an ISVP generated from a reovirus carrying a genetically-engineered or a spontaneously generated mutation affecting at least the sigma3 polypeptide. The ISVPs described herein are stable and can be propagated as ISVPs for multiple (e.g., more than one, e.g., 2, 3, 4, 5, 10, 20, 50, or more) passages.

The mutant reoviruses described herein, produced via a genetically-engineered ISVP or via a plasmid-based reverse genetic system, can be cultured in, for example, human neoplastic cells or L929 mouse fibroblast cells. Mutant reoviruses disclosed herein can be cultured in cells that are only permissive to reovirus strains lacking the sigma3 polypeptide. Using such cell lines to passage the mutant reoviruses described herein can allow for selection of the mutants and also can be used to reduce or prevent reversions of the mutation(s).

Mutant reoviruses can be purified using standard methodology. See, for example, Schiff et al., "Orthoreoviruses and Their Replication," Ch 52, in Fields Virology, Knipe & Howley, eds., 2006, Lippincott Williams & Wilkins; Smith et al., 1969, Virology, 39(4):791-810; and U.S. Patent Nos. 7,186,542; 7,049,127; 6,808,916; and 6,528,305. Affinity chromatography can be used with, for example, an antibody directed toward the sigma3 polypeptide to remove reoviruses that contain the sigma3 polypeptide and to allow the mutants lacking the sigma3 polypeptide to flow-through. As used herein, purified mutant reoviruses refer to reoviruses that have been separated from cellular components that naturally accompany them. Typically, reoviruses are considered purified when they are at least 70% (e.g., at least 75%, 80%, 85%, 90%, 95%, or 99%) by dry weight, free from the proteins and other cellular components with which they are naturally associated.

The mutant reoviruses described herein exhibit increased infectivity and/or decreased immunogenicity as compared to a non-mutant reovirus (e.g., a control reovirus) and can be selected on the basis of such traits. Increased infectivity can be evidenced by an increase in the range of neoplastic cells and/or the number of cells that are infected by a mutant reovirus compared to a reovirus that expresses a functional sigma3 polypeptide (e.g., an intact virion; e.g., a wild type reovirus). Decreased immunogenicity of mutant reoviruses can be evidenced by the inability of such mutant reoviruses to induce a significant immune response in the subject. The mutant reoviruses described herein also can be screened and selected for other desirable traits including, but not limited to, a faster rate of replication; a faster rate of packaging; the ability to induce apoptosis; the ability to affect lysis in and effectively kill human neoplastic cells lines; the ability to release effective tumor epitopes; interaction with standard chemotherapies; and an increased number of viral progeny. Additionally, mutant reoviruses can be selected for the ability to lytically infect a neoplastic cell (e.g., a mammalian cell having an active Ras pathway). See, for example, U.S. Patent No. 7,052,832.

### Methods of Using Reoviruses Having Modified Sequences

As described previously (see, for example, U.S. Patent Nos. 6,110,461; 6,136,307; 6,261,555; 6,344,195; 6,576,234; and 6,811,775), reoviruses use a host cell's Ras pathway machinery to downregulate double-stranded RNA-activated protein kinase (PKR) and thus replication in the cell. Based upon these discoveries, methods have been developed for using reoviruses to treat proliferative disorders in mammals such as mice, dogs, cats, sheep, goats, cows, horses, pigs, non-human primates, and humans. The mutant reoviruses described herein can be used to treat a proliferative disorder in a subject.

A proliferative disorder is any cellular disorder in which the cells proliferate more rapidly than normal tissue growth. Thus a proliferating cell is a cell that is proliferating more rapidly than normal cells. A proliferative disorder includes, but is not limited to, neoplasms, which are also referred to as tumors. A neoplasm can include, but is not limited to, pancreatic cancer, breast cancer, brain cancer (e.g., glioblastoma), lung cancer, prostate cancer, colorectal cancer, thyroid cancer, renal cancer, adrenal cancer, liver cancer, neurofibromatosis 1, and leukemia. A neoplasm can be a solid neoplasm (e.g., sarcoma or carcinoma) or a cancerous growth affecting the hematopoietic system (e.g., lymphoma or leukemia). Other proliferative disorders include, but are not limited to neurofibromatosis.

Generally, in proliferating disorders for which reovirus is used as a treatment, at least some of the proliferating cells may have a mutation in which the Ras gene (or an element of the Ras signaling pathway) is activated, either directly (e.g., by an activating mutation in Ras) or indirectly (e.g., by activation of an upstream or downstream element in the Ras pathway). Activation of an upstream element in the Ras pathway includes, for example, transformation with epidermal growth factor receptor (EGFR) or Sos. See, for example, Wiessmuller & Wittinghofer, 1994, Cellular Signaling, 6(3):247-267; and Barbacid, 1987, Ann. Rev. Biochem., 56, 779-827. Activation of a downstream element in the Ras pathway includes, for example, mutation within B-Raf. See, for example, Brose et al. (2002, Cancer Res., 62:6997-7000). In addition, the reovirus is useful for treating proliferative disorders caused by mutations or dysregulation of PKR. See, for example, Strong et al. (1998, EMBO J., 17:3351-62).

The mutant reoviruses described herein can be administered to a mammal that has a proliferative disorder. As used herein, administration refers to delivery of a mutant reovirus (e.g., an ISVP) such that the mutant reovirus contacts the proliferating cells. The route by which a mutant reovirus is administered will depend on the type of disorder and the location of the proliferating cells. A wide variety of administration routes can be employed, and the following are provided simply by way of example and are not meant to be limiting in any way.

For a solid neoplasm, for example, a mutant reovirus can be administered by methods that include direct injection into the tumor or systemic administration (e.g., intravenously) by injection, infusion or the like For a hematopoietic neoplasm, for example, a mutant reovirus can be administered intravenously or intravascularly. For metastatic neoplasms or neoplasms such as brain tumors, for example, a mutant reovirus can be administered in a manner such that it is transported systemically through the body (e.g., intrathecally, intravenously or intramuscularly). A mutant reovirus also can be administered subcutaneously, intraperitoneally (e.g., for ovarian neoplasms), topically (e.g., for melanomas), orally (e.g., for oral or esophageal neoplasms), rectally (e.g., for colorectal neoplasms), vaginally (e.g., for cervical or vaginal neoplasms), nasally or by inhalation spray (e.g., for lung neoplasms). A mutant reovirus can be administered by more than one route and/or to more than one location in a subject.

A mutant reovirus as disclosed herein is administered in an amount that is sufficient to treat the proliferative disorder (e.g., an effective amount). A proliferative disorder is treated when administration of a mutant reovirus to proliferating cells affects lysis (e.g., oncolysis) of the cells, resulting in a reduction in the number of proliferating cells, a reduction in the size of a neoplasm, and/or a reduction in or elimination of symptoms (e.g., pain) associated with the proliferating disorder. As used herein, the term oncolysis means at least 10% of the proliferating cells are lysed (e.g., at least about 20%, 30%, 40%, 50%, or 75% of the cells are lysed). The percentage of lysis can be determined, for example, by measuring the reduction in the size of a neoplasm or in the number of proliferating cells in a mammal, or by measuring the amount of lysis of cells *in vitro* (e.g., from a biopsy of the proliferating cells).

An effective amount of a mutant reovirus will be determined on an individual basis and may be based, at least in part, on the particular mutant reovirus used; the individual's size, age, gender; and the size and other characteristics of the proliferating cells. For example, for treatment of a human, approximately 10³ to 10¹² plaque forming units (PFU) of a mutant reovirus can be used, depending on the type, size and number of proliferating cells or neoplasms present. The effective amount can be, for example, from about 1.0 PFU/kg body weight to about 10¹⁵ PFU/kg body weight (e.g., from about 10² PFU/kg body weight to about 10¹³ PFU/kg body weight). A mutant reovirus can be administered in a single dose or in multiple doses (e.g., two, three, four, six, or more doses). Multiple doses can be administered concurrently or consecutively (e.g., over a period of days or weeks). Treatment with a mutant reovirus may last from several days to several months or until diminution of the disease is achieved.

It is contemplated that a mutant reovirus as disclosed herein can be administered in conjunction with surgery or removal of proliferating cells (e.g., a neoplasm). It also is contemplated that a mutant reovirus can be administered in conjunction with or in addition to radiation therapy. It is further contemplated that a mutant reovirus can be administered in conjunction with or in addition to anticancer compounds, chemotherapeutic agents, and/or immunosuppressive agents. Such agents include, but are not limited to, 5-fluorouracil, mitomycin C, methotrexate, hydroxyurea, gemcitabine, cyclophosphamide, dacarbazine, mitoxantrone, anthracyclins (e.g., epirubicin and doxurubicin), tubulin-stabilizing agents (e.g., vinca alkaloids), antibodies to receptors such as HERCEPTIN® (Genentech, South San Francisco, CA), etoposide and pregnasome, platinum compounds such as carboplatin and cisplatin, taxanes such as TAXOL® (Bristol-Myers Squibb, New York, NY) and TAXOTERE® (Rhone-Poulenc Rorer SA, Antony, France), hormone therapies such as tamoxifen and anti-estrogens, interferons, aromatase inhibitors, progestational agents and LHRH analogs. It is also contemplated that a mutant reovirus as disclosed herein can be administered in conjunction with or in addition to vascular permeability agents such as, without limitation, IL-2, TNF-alpha, VEGF, AVASTIN® (Genentech), and relaxin.

### Pharmaceutical Compositions

Pharmaceutical compositions that include a mutant reovirus as described herein are provided. See, for example, U.S. Patent No. 6,576,234. In addition to one or more mutant reoviruses, a pharmaceutical composition typically includes a pharmaceutically acceptable carrier. A pharmaceutically acceptable carrier can be a solid, semi-solid, or liquid material that can act as a vehicle, carrier or medium for the mutant reovirus. Thus, compositions containing a mutant reovirus can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

Some examples of suitable carriers include phosphate-buffered saline or another physiologically acceptable buffer, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. A pharmaceutical composition additionally can include, without limitation, lubricating agents such as talc, magnesium stearate, and mineral oil; wetting agents; emulsifying and suspending agents; preserving agents such as methyl- and propylhydroxy-benzoates; sweetening agents; and flavoring agents. Pharmaceutical compositions can be formulated to provide quick, sustained or delayed release of a mutant reovirus after administration by employing procedures known in the art. In addition to the representative formulations described below, other suitable formulations for use in a pharmaceutical composition can be found in Remington: The Science and Practice of Pharmacy (2003, Gennaro & Gennaro, eds., Lippincott Williams & Wilkens).

For preparing solid compositions such as tablets, a mutant reovirus can be mixed with a pharmaceutical carrier to form a solid composition. Optionally, tablets or pills can be coated or otherwise compounded to provide a dosage form affording the advantage of prolonged action. For example, a tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Liquid formulations that include a mutant reovirus for oral administration or for injection generally include aqueous solutions, suitably flavored syrups, aqueous or oil suspensions, and flavored emulsions with edible oils such as corn oil, cottonseed oil, sesame oil, coconut oil, or peanut oil, as well as elixirs and similar pharmaceutical vehicles.

Compositions for inhalation or insufflatiori include solutions and suspensions in pharmaceutically acceptable, aqueous or organic solvents, or mixtures thereof, and powders. These liquid or solid compositions may contain suitable pharmaceutically acceptable excipients as described herein. Such compositions can be administered by the oral or nasal respiratory route for local or systemic effect. Compositions in pharmaceutically acceptable solvents may be nebulized by use of inert gases. Nebulized solutions may be inhaled directly from the nebulizing device or the nebulizing device may be attached to a face mask tent or intermittent positive pressure breathing machine. Solution, suspension, or powder compositions may be administered, orally or nasally, from devices which deliver the formulation in an appropriate manner.

Another formulation that can be employed in the methods of the present disclosure employs transdermal delivery devices (e.g., patches). Such transdermal patches may be used to provide continuous or discontinuous infusion of a mutant reovirus as described herein. The construction and use of transdermal patches for the delivery of pharmaceutical agents is well known in the art. See, for example, U.S. Patent No. 5,023,252. Such patches can be constructed for continuous, pulsatile, or on-demand delivery of mutant reoviruses.

Mutant reoviruses can, if necessary, be coated in a liposome or micelle to reduce or prevent an immune response in a mammal that has developed immunity toward a reovirus. Such compositions are referred to as immunoprotected reoviruses. See, for example, U.S. Patent Nos. 6,565,831 and 7,014,847. In addition, a mutant reovirus as disclosed herein (e.g., one that lacks or is deficient in sigma3 polypeptide or function) can be proteolytically treated with an enzyme to remove or partially remove any of the other outer capsid proteins present.

Mutant reoviruses or a pharmaceutical composition comprising such mutant reoviruses can be packaged into a kit. It is contemplated that a kit also can include one or more chemotherapeutic agents, one or more immunosuppressive agents, and/or one or more anti-antireovirus antibodies. A pharmaceutical composition can be formulated in a unit dosage form. The term "unit dosage forms" refers to physically discrete units suitable as unitary dosages for human subjects and other mammals, each unit containing a predetermined quantity of a mutant reovirus calculated to produce the desired therapeutic effect in association with a suitable pharmaceutically acceptable carrier.

In accordance with the present disclosure, there may be employed conventional molecular biology, microbiology, biochemical, and recombinant DNA techniques within the skill of the art. Such techniques are explained fully in the literature. The methods and compositions of matter will be further described in the following examples, which do not limit the scope of the invention described in the claims.

### EXAMPLES

### Example 1-Generating a Mutant Reovirus

A construct that expresses a mutant form of the S4 gene under the control of a constitutive or an inducible promoter is generated such that the plus-strand RNA is transcribed. In one example, the start codon for translation of the S4 gene is mutated by deletion, insertion or substitution. A reovirus-susceptible cell line then is transformed with the construct.

The transformed cell line is infected with a reovirus and, if necessary, expression of the S4 gene is induced. The plus-strand S4 RNA containing the mutation is a substrate for the viral RNA-dependent RNA polymerase, which generates double-stranded mutant S4 RNAs that are packaged but are non-functional, at least with respect to incorporation of the encoded sigma3 polypeptide into a capsid.

Progeny virus from the transformed cell include both wild-type reovirus that express the sigma3 polypeptide as well as mutant reovirus that do not express the sigma3 polypeptide. A mutant reovirus that does not express the sigma3 polypeptide is referred to occasionally as a naked reovirus.

The naked viruses are selected, for example, using a plaque titration assay with a cell line that has demonstrated a block in the uncoating step. In such a cell line, the wild-type virus is not able to effectively replicate. The largest plaque is selected and expanded. Alternatively, the wild type reovirus is purified out using affinity chromatography with antibodies directed against the sigma3 polypeptide. The presence or absence of the sigma3 polypeptide is determined by Western blotting and/or immunoprecipitation.

It is to be understood that while the materials and methods have been described in conjunction with the detailed description thereof, the foregoing description is intended to illustrate and not limit the scope of the materials and methods, which is defined by the scope of the appended claims. Other aspects, advantages, and modifications are within the scope of the following claims.

Disclosed are methods and compositions that can be used for, can be used in conjunction with, can be used in preparation for, or are products of the disclosed methods and compositions. These and other materials are disclosed herein, and it is understood that combinations, subsets, interactions, groups, etc. of these methods and compositions are disclosed. That is, while specific reference to each various individual and collective combinations and permutations of these compositions and methods may not be explicitly disclosed, each is specifically contemplated and described herein. For example, if a particular mutation in a reovirus or a particular method is disclosed and discussed and a number of mutations that can be made to the reovirus or modifications that can be made to the methods are discussed, each and every combination and permutation of the reovirus and the methods are specifically contemplated unless specifically indicated to the contrary. Likewise, any subset or combination of these is also specifically contemplated and disclosed.

## Claims

1. An isolated, genetically-engineered reovirus infectious subviral particle (ISVP), wherein said ISVP comprises a nucleic acid comprising a mutation and wherein the mutation results in lack of expression of a sigma3 polypeptide.

2. The reovirus ISVP of claim 1, wherein the mutation is in a nucleic acid encoding the sigma3 polypeptide or in a nucleic acid that regulates expression of the sigma3 polypeptide.

3. The reovirus ISVP of claim 2, wherein said nucleic acid is the S4 gene.

4. The reovirus ISVP of claim 1, wherein the mutation is a substitution, or wherein the mutation is an insertion or deletion of one or more nucleotides.

5. The reovirus ISVP of claim 1, further comprising one or more further mutations, optionally selected from the group consisting of:
a mutation that reduces expression of a mul polypeptide, essentially eliminates expression of a mul polypeptide, or results in an absence of a functional mu1 polypeptide;
a mutation that reduces expression of a lambda2 polypeptide, essentially eliminates expression of a lambda2 polypeptide, or results in an absence of a function lambda2 polypeptide; and/or
a mutation that reduces expression of a sigmal polypeptide, essentially eliminates expression of a sigmal polypeptide, or results in an absence of a functional sigmal polypeptide.

6. A method of making a reovirus having increased infectivity, comprising
genetically-engineering a mutation in a first reovirus to generate a mutant reovirus, wherein said mutant reovirus lacks expression of a sigma3 polypeptide;
culturing said mutant reovirus; and
isolating ISVPs to thereby obtain a mutant reovirus ISVP.

7. The reovirus ISVP of claim 1, for use in a method of treating a proliferative disorder in a subject.

8. The reovirus ISVP for use as claimed in claim 7, wherein the method further comprises at least one of the procedures selected from the group consisting of surgery, chemotherapy, radiation therapy, and immunosuppressive therapy.

9. A pharmaceutical composition comprising the reovirus ISVP of claim 1.

10. The pharmaceutical composition of claim 9, further comprising one or more chemotherapeutic agents and/or one or more immunosuppressive agents.

## Patentansprüche

1. Isoliertes, genetisch verändertes infektiöses subvirales Reovirus-Teilchen (ISVP), wobei das ISVP eine Nukleinsäure umfasst, umfassend eine Mutation, und wobei die Mutation zu fehlender Expression eines Sigma3-Polypeptids führt.

2. Reovirus-ISVP gemäß Anspruch 1, wobei die Mutation in einer Nukleinsäure liegt, die das Sigma3-Polypeptid kodiert, oder in einer Nukleinsäure, welche die Expression des Sigma3-Polypeptids regelt.

3. Reovirus-ISVP gemäß Anspruch 2, wobei die Nukleinsäure das S4-Gen ist.

4. Reovirus-ISVP gemäß Anspruch 1, wobei die Mutation eine Substitution ist, oder wobei die Mutation eine Insertion oder Entfernung von einem oder mehreren Nukleotiden ist.

5. Reovirus-ISVP gemäß Anspruch 1, zudem umfassend eine oder mehrere Mutationen, wahlweise ausgewählt aus der Gruppe
eine Mutation, welche die Expression eines My1-Polypeptids verringert, im Wesentlichen die Expression eines My1-Polypeptids ausschaltet, oder zu einer Abwesenheit eines funktionalen My1-Polypeptids führt;
eine Mutation, welche die Expression eines Lambda2-Polypeptids verringert, im Wesentlichen die Expression eines Lambda2-Polypeptids ausschaltet, oder zu einer Abwesenheit eines funktionalen Lambda2-Polypeptids führt;
eine Mutation, welche die Expression eines Sigma1-Polypeptids verringert, im Wesentlichen die Expression eines Sigma1-Polypeptids ausschaltet, oder zu einer Abwesenheit eines funktionalen Sigma1-Polypeptids führt.

6. Herstellungsverfahren für ein Reovirus mit erhöhter Infektiosität, umfassend
genetische Herstellung einer Mutation in einem ersten Reovirus zum Erzeugen eines mutierten Reovirus, wobei dem mutierten Reovirus Expression eines Sigma3-Polypeptids fehlt;
Züchten des mutierten Reovirus; und
Isolieren von ISVPs, um so mutierte Reovirus-ISVPs zu erhalten.

7. Reovirus-ISVP gemäß Anspruch 1, zur Verwendung in einem Behandlungsverfahren für eine proliferative Krankheit in einem Subjekte.

8. Reovirus-ISVP zur Verwendung, wie in Anspruch 7 beansprucht, wobei das Verfahren zudem umfasst mindestens eine der Prozeduren, ausgewählt aus der Gruppe Operation, Chemotherapie, Radiotherapie und immunosuppressive Therapie.

9. Pharmazeutische Zusammensetzung, umfassend das Reovirus-ISVP aus Anspruch 1.

10. Pharmazeutische Zusammensetzung gemäß Anspruch 9, zudem umfassend ein oder mehrere chemotherapeutische Mittel und/oder ein oder mehrere immunosuppressive Mittel.

## Revendications

1. Une particule sous-virale infectieuse de réovirus (ISVP) génétiquement modifiée isolée, dans laquelle ledit ISVP comprend un acide nucléique comprenant une mutation et dans laquelle la mutation résulte en l'absence d'expression d'un polypeptide sigma3.

2. Le réovirus ISVP de la revendication 1, dans lequel la mutation est dans un acide nucléique codant le polypeptide sigma3 ou dans un acide nucléique qui régule l'expression du polypeptide sigma3.

3. Le réovirus ISVP de la revendication 2, dans lequel ledit acide nucléique est le gène S4.

4. Le réovirus ISVP de la revendication 1, dans lequel la mutation est une substitution ou dans lequel la mutation est une insertion ou une suppression d'un ou plusieurs nucléotides.

5. Le réovirus ISVP de la revendication 1, comprenant en outre une ou plusieurs autres mutations, éventuellement sélectionnées parmi le groupe composé de :
une mutation qui réduit l'expression d'un polypeptide mu1, élimine essentiellement l'expression d'un polypeptide mu1 ou les résultats en l'absence d'un polypeptide mu1 fonctionnel ;
une mutation qui réduit l'expression d'un polypeptide lambda2, élimine essentiellement l'expression d'un polypeptide lambda2 ou les résultats en l'absence d'un polypeptide lambda2 fonctionnel ; et/ou
une mutation qui réduit l'expression d'un polypeptide sigma1, élimine essentiellement l'expression d'un polypeptide sigmal ou les résultats en l'absence d'un polypeptide sigmal fonctionnel.

6. Une méthode de fabrication d'un réovirus présentant une infectiosité accrue, comprenant
la modification génétique d'une mutation dans un premier réovirus pour générer un réovirus mutant, dans lequel ledit réovirus mutant ne possède pas l'expression d'un polypeptide sigma3 ;
la culture dudit réovirus mutant ; et
l'isolation des ISVP pour obtenir ainsi un ISVP de réovirus mutant

7. L'ISVP de réovirus de la revendication 1, destinée à une utilisation dans une méthode de traitement d'un trouble prolifératif chez un sujet.

8. L'ISVP de réovirus destinée à une utilisation selon la revendication 7, dans lequel la méthode comprend en outre au moins l'une des procédures sélectionnées parmi le groupe composé de la chirurgie, la chimiothérapie, la radiothérapie et la thérapie immunosuppressive.

9. Une composition pharmaceutique comprenant l'ISVP de réovirus de la revendication 1.

10. La composition pharmaceutique de la revendication 9, comprenant en outre un ou plusieurs agents chimiothérapeutiques et/ou un ou plusieurs agents immunosuppresseurs.
